# EUROPÄISCHE PATENTSCHRIFT

(11) **EP 1 991 154 B1**
(45) Veröffentlichungstag und Bekanntmachung des Hinweises auf die Patenterteilung: **23.09.2009**
(21) Anmeldenummer: 07701927.1
(22) Anmeldetag: 08.03.2007
(51) Int. Cl.: A61C 3/03, A61C 17/20

(54) **UMLENKUNG VON MECHANISCHEN OSZILLATIONEN**
DIVERSION OF MECHANICAL OSCILLATIONS
DÉVIATION D'OSCILLATIONS MÉCANIQUES

(30) Priorität: 09.03.2006 CH 376062006
(43) Veröffentlichungstag der Anmeldung: 19.11.2008
(73) Patentinhaber: Woodwelding AG, 6304 Zug (CH)
(72) Erfinder: MAYER, Jörg, CH-5702 Niederlenz (CH); AESCHLIMANN, Marcel, CH-2514 Ligerz (CH); TORRIANI, Laurent, CH-2516 Lamboing (CH); RUSCH, Christoph, Ch-2502 Biel (CH); GILLIERON, Stéphane, CH-2000 Neuchâtel (CH)
(74) Vertreter: Frei Patent Attorneys
(86) Internationale Anmeldenummer: PCT/CH2007/000129
(87) Internationale Veröffentlichungsnummer: WO 2007/101362

(56) Entgegenhaltungen:
- EP-A- 0 535 542
- EP-A- 1 530 953
- DE-U1- 20 113 692
- US-A- 4 992 048
- US-A- 5 100 321
- US-A- 5 899 693
- US-A1- 2003 157 458

## Beschreibung

Die Erfindung betrifft die Applikation von mechanischen Oszillationen, bspw. Ultraschalloszillationen, in Situationen, in denen beschränkte Platzverhältnisse die Bewegungsfreiheit einschränken. Sie betrifft speziell eine Vorrichtung zum Umlenken von mechanischen Schwingungen, insbesondere eine Sonotrode oder ein Kopplungsstück.

Ultraschall-Bearbeitungsgeräte werden heute vermehrt auch im medizinischen Sektor, unter anderem in der Zahnmedizin angewandt. Ein Beispiel für eine Anwendung von Ultraschallgeräten in der Medizin, insbesondere Zahnmedizin, ist ein neu entwickeltes Verfahren zum Verankern von Implantaten und Präparaten in porösem Gewebe. Das Verfahren ist beispielsweise in den Schriften WO 02/069'817, WO 2004/017'927 und WO 2004/017 857 beschrieben.

Ultraschall-Bearbeitungsgeräte für den medizinischen Gebrauch haben praktischerweise oft eine längliche Form mit Handgriff, so dass sie ähnlich einem Zahnarztbohrer in der Art eines Handwerkzeuges verwendet werden können. Der beispielsweise piezoelektrische Schwingungsanreger regt eine Sonotrode zu longitudinalen Schwingungen an, die diese an ein Werkzeug oder Werkstück überträgt. Aufgrund der länglichen Form des Geräts ist aber die Arbeit an schwer zugänglichen Orten erschwert.

Aus der EP 0 594 541 ist eine Sonotrode bekannt, die als ringförmiger Biegeschwinger ausgebildet ist. Die Sonotrode schwingt um vier Knotenpunkte, was eine Umlenkung der Schwingung um einen Winkel von 90° möglich macht. Die Sonotrode kann auch um mehr als vier Knoten schwingen, so dass eine Umlenkung um andere ganzzahlige Teiler von 360° möglich ist, beispielsweise um 120°. Nachteilig bei der Sonotrode der EP 0 594 541 ist, dass sie aufgrund des ringförmigen Aufbaus relativ viel Platz beansprucht. Ausserdem wird nur ungefähr die Hälfte der in die Sonotrode eingekoppelten Leistung auch ins Werkzeug oder Werkstück ausgekoppelt. Noch ein Nachteil ist, dass eine Umlenkung nur um Winkel möglich ist, die ein ganzzahliger Teiler von 360° sind. Als weiterer Nachteil sind aufgrund der ringförmigen Konstruktion nur kleine Schwingungsamplituden möglich.

Ein dazu alternatives Vorgehen für das Umlenken von Ultraschall ist in der US-Patentschrift 6,139,320 beschrieben. Ein Umlenkkanal enthält eine Flüssigkeit, durch die die longitudinalen Schwingungen entsprechend der Form des Umlenkkanals umgeleitet werden können. Nachteilig dabei ist, dass aufgrund einer gewissen Kompressibilität der Flüssigkeit Energie verloren geht und sich die Flüssigkeit dabei erwärmt.

Es ist demnach eine Aufgabe der Erfindung, eine Lösungen für das Umlenken von mechanischen Schwingungen zur Verfügung zu stellen, welche Nachteile von Ansätzen gemäss dem Stand der Technik überwindet und welche insbesondere geeignet für den Einsatz bei beschränkten Platzverhältnissen sein soll.

Besonders bevorzugt sind Lösungen, die ein Umlenken zwischen einer Schwingungs-Aufnahmestelle und einer Schwingungs-Abgabestelle um ungefähr 100°-130° ermöglichen, weil bei diesen Winkeln die Arbeit an schwer zugänglichen Stellen oft besonders leicht fällt.

Diese Aufgabe wird gelöst durch die Erfindung, wie sie in den Patentansprüchen definiert ist.

Eine erfindungsgemässe Vorrichtung zum Umlenken von mechanischen Schwingungen, ist an einer Schwingungs-Aufnahmestelle längs einer ersten Achse in Schwingung versetzbar und überträgt eine solche Schwingung in eine Schwingung längs einer zweiten Achse an einer Schwingungs-Abgabestelle, wobei die erste und die zweite Achse einen Winkel zueinander bilden. An der Schwingungs-Aufnahmestelle ist die Vorrichtung zum Anschliessen eins Schwingungsanregers, an der Schwingungs-Abgabeststelle zum Anschliessen eines Werkzeugs, Werkstücks oder Zwischenstücks ausgebildet. Die Vorrichtung zeichnet sich im Wesentlichen dadurch aus, dass sie ein längliches, zwischen zwei Enden gebogenes Schwingelement aufweist, an dem ein Einkoppelpunkt und ein Auskoppelpunkt angeordnet sind, wobei die Vorrichtung so ausgebildet ist, dass das Schwingelement am Einkoppelpunkt und am Auskoppelpunkt transversal schwingt, wenn die Schwingungs-Aufnahmestelle mit einer Schwingung beaufschlagt wird.

Die mechanischen Schwingungen sind bspw. Ultraschallschwingungen.

Die erfindungsgemässe Vorrichtung wirkt gemäss obiger Lehre also als Sonotrode, wobei der Begriff "Sonotrode" nicht bedeutet, dass die Vorrichtung direkt am Werkzeug oder Werkstück angreifen muss; vielmehr kann auch ein Zwischenstück vorhanden sein, das die Schwingungen von der Sonotrode an ein Werkzeug oder Werkstück oder ein weiteres Zwischenstück weiter überträgt.

Dadurch, dass die Schwingungen vom Schwingelement transversal abgegriffen werden, wirkt das Schwingelement in der Art eines "Hammers", der einen in Bezug auf die Schwingelement-Achse seitliches Werkzeug, Werkstück oder Zwischenstück mit den Schwingungen beaufschlägt.

Es hat sich gezeigt, dass es mit erfindungsgemässen Vorgehen möglich ist, am Auskoppelpunkt Schwingungen mit einer guten Linearität zu erzeugen, d.h. Schwingungen deren Komponente quer zur gewünschten (in Bezug auf das Schwingelement transversalen) Schwingungsrichtung sehr klein ist. D.h. es ist möglich Schwingungen zu erzeugen, die entlang einer sehr langgestreckt-elliptischen Bahn verlaufen, so dass in sehr guter Näherung von durch Schwingungen entlang einer Gerade ausgegangen werden kann.

Zwischen der Vorrichtung und dem Werkzeug, Werkstück oder Zwischenstück - und auch zwischen dem Schwingungsanreger und der Vorrichtung - kann eine feste, lösbare Kupplung vorhanden sein. Eine solche hat zur Folge, dass das Werkzeug, Werkstück oder Zwischenstück die Schwingung an der Schwingungs-Abgabestelle voll mitmacht, d.h. dass jeweils beide Halbwellen übertragen werden. Als Alternative kann die Übertragung auch durch einen nur losen Kontakt erfolgen, wobei dann nur Druck- und keine Zugkräfte übertragbar sind.

Die länglich-gebogene Ausgestaltung des Schwingelementes bedeutet, dass eine Längsachse und damit eine longitudinale Richtung und transversale Richtungen definiert sind. Das Schwingelement kann bspw. die Form eines gebogenen Stabes beliebigen Querschnitts aufweisen, wobei die Querschnittsfläche des Stabes über seine Länge nicht konstant sein muss. Der Ein- und der Auskoppelpunkt können sich je in der Nähe eines Endes des Stabes befinden. Am Stab können zusätzliche Elemente befestigt sein, bspw. Massen, mit denen das Amplitudenverhältnis zwischen Schwingungen am Einkoppel- und am Auskoppelpunkt beeinflussbar ist.

Eine der Erfindung zu Grunde liegende Erkenntnis ist, dass die transversalen Schwingungen eines solchen Schwingelementes mit einem länglichen, gebogenen Abschnitt dafür geeignet sind, mechanische Vibrationen um verschiedene, durch die Wahl Geometrie des Schwingelementes fast frei wählbare Winkel umzulenken.

Bevorzugt verläuft das Schwingelement in einer Ebene und schwingt in dieser Ebene. Der Umlenk-Winkel wird bestimmt durch die Biegung des Schwingelementes in der Ebene sowie durch die Lage des Einkoppel- und des Auskoppelpunkts: Durch die Biegung in der Ebene wird eine Innen- und eine Aussenseite definiert. Falls der Auskoppelpunkt auf der Aussenseite liegt, entspricht der Umlenkwinkel dem Biegewinkel, also dem Winkel zwischen der Schwingelement-Längsachse am Einkoppel- und am Auskoppelpunkt. Falls sich hingegen der Auskoppelpunkt auf der Innenseite befindet ist der Umlenkwinkel 180° minus dem Biegewinkel.

Unter Umständen - im Allgemeinen weniger bevorzugt - ist auch ein Abgreifen der Schwingung am Schwingelement durch ein Objekt (Werkzeug, Werkstück oder Zwischenstück) möglich, welches stirnseitig am Schwingelement angebracht ist. Ein "aussenseitiger" Auskoppelpunkt bedeutet dann, dass das Werkzeug, Werkstück oder Zwischenstück während der Bearbeitung im Bezug auf die Biegung des Schwingelements aussen liegt, ein "innenseitiger Auskoppelpunkt umgekehrt dass es innen liegt.

Durch die Wahl von Biegewinkel und Ein- und Auskoppelpunkten wird ein praktisch beliebiger Umlenkwinkel bei einer grossen Variabilität von äusseren Abmessungen ermöglicht. Im Unterschied zum Stand der Technik können seine äusseren Abmessungen für gegebene Umlenkwinkel, Frequenz und Leistung sowie ein gegebenes Amplitudenverhältnis innerhalb gewisser Randbedingungen gewählt werden, bspw. durch Wahl der Massenschwerpunkte von zwei Schwingelement-Hälften (entsprechend zwei Schwingelement-Armen), der Wahl von Einkoppel- und Auskoppelpunkt (innen/aussen) etc. Das Schwingelement kann bspw. zum Werkzeug, Werkstück oder Zwischenstück "hin" oder "davon weg" gebogen sein.

Besonders bevorzugt ist der Fall, in welchem der Biegewinkel mehr als 90° beträgt und sowohl Ein- als auch Auskoppelpunkt auf der Aussenseite liegen. Das ist aus Platzgründen für die meisten Anwendungsgeometrien besonders günstig; ein Biegewinkel von mehr als 90° hat sich auch schwingungstechnisch als besonders günstig herausgestellt.

Der bevorzugte Umlenkwinkel ist zwischen 100° und ca. 130°, besonders bevorzugt 110° bis 120°. Die Erfindung erlaubt auch Umlenkwinkel, die keine ganzzahligen Teiler von 360° sind.

Das Schwingelement kann bspw. eine Form aufweisen, die im Wesentlichen einem Kreisbogen-Ausschnitt entspricht, es kann V-förmig, Ω-förmig, hakenförmig etc. sein. In Ausführungsformen, bei denen nicht das ganze Schwingelement gleichmässig gekrümmt ist, sondern bei denen die Krümmung in einem Krümmungsbereich besonders stark ist (bspw. bei der V- oder Ω-form), müssen nicht beide an den Krümmungsbereich anschliessenden Arme gleich lang sein, sondern das Schwingelement kann eine asymmetrische Form aufweisen. Auf jeden Fall bildet das Schwingelement keinen geschlossenen Ring sondern quasi einen offenen Ring gleichmässiger oder ungleichmässiger Krümmung. Das ist in mehrerer Hinsicht vorteilhaft. Einerseits sind aufgrund der nicht-geschlossenen Konstruktion viel grössere Amplituden möglich als bei einer ringförmigen Sonotrode. Andererseits gibt es bei einer ringförmigen Sonotrode zwischen Knoten der Schwingung mehrere Abschnitte, die schwingen. In der Anwendung wird aber im Allgemeinen nicht die Schwingung jedes solchen Abschnittes benutzt. Die Leistung, die zum In-Schwingung-Versetzen eines nicht benutzten Abschnittes benötigt wird, geht also verloren. Aus diesem Grund ist der Wirkungsgrad der erfindungsgemässen Konstruktion im Allgemeinen höher als einer ringförmigen Sonotrode gemäss dem Stand der Technik. Schliesslich ist die nicht-ringförmige Konstruktion auch vorteilhaft, weil sich wie erwähnt praktisch beliebige Umlenkwinkel realisieren lassen und der Platzbedarf geringer ist.

Der Querschnitt des Schwingelementes muss nicht homogen sein, sondern kann im Gegenteil variieren. Solche Variationen des Querschnitts oder der Armlängen können ebenso wie die Materialwahl, Querschnittsprofile, Form des Schwingelementes und Massenverteilung sowie die Positionen des Einkoppelpunktes und des Auskoppelpunktes und eventuelle Ausgestaltung von Gelenken, Zusatzelementen etc. dazu benutzt werden, die Form, Eigenfrequenzen und Amplitudenverhältnisse der Eigenschwingung zu beeinflussen. Bspw. kann eine Amplitudenverstärkung oder -abschwächung durch eine Wahl der Massenverteilung zwischen zwei Armen bewirkt werden.

Das Schwingelement kann bspw. ähnlich der beiden Arme einer Stimmgabel ausgebildet sein, wobei die beiden Arme des Schwingelementes im Unterschied zu Stimmgabeln, die für das Stimmen von Musikinstrumenten verwendet werden, nicht parallel zueinander verlaufen, sondern in einem der Aufgabe angepassten Winkel.

Das Schwingelement ist am Einkoppelpunkt beispielsweise elastisch mit einem Schwingungsanreger des Schwingungen erzeugenden Geräts - bspw. Ultraschallgeräts - verbunden. Eine elastische Verbindung bedeutet, dass die Steifigkeit der Verbindung (genauer: deren Federkonstante) kleiner ist als die Steifigkeit des Schwingelementes selbst und auch kleiner als diejenige anderer Komponenten der Vorrichtung.

Eine elastische Verbindung zwischen dem Schwingungsanreger und dem Schwingelement erlaubt, dass sich eine der Eigenschwingung entsprechende Schwingung mit der Anregungsfrequenz ausbildet, ohne dass sich der Massenschwerpunkt des Schwingelementes - also sozusagen das Schwingelement als Ganzes - während einer Auslenkung wesentlich verschieben müsste. Das wirkt sich positiv auf den Wirkungsgrad aus, da weniger grosse Massen beschleunigt werden müssen.

Die Verbindung zwischen dem Schwingungsanreger und dem Schwingelement kann gemäss einer ersten bevorzugten Ausführungsform ein Gelenk, bspw. elastisches Gelenk aufweisen. Dieses wirkt bspw. scharnierartig, d.h. es erlaubt Kippbewegungen in einer Richtung, nicht aber in der Richtung senkrecht dazu. Das Gelenk kann am stirnseitigen Ende des Schwingelementes oder seitlich (lateral) angebracht sein. Es ist vorzugsweise einstückig mit dem Schwingelement und einem Befestigungselement ausgebildet und kann die Form einer Einschnürung haben, durch welche die Materialdicke lokal so reduziert ist, dass das Gelenk Verschwenkungen in der Schwingelement-Ebene zulässt, nicht jedoch senkrecht dazu.

Das Befestigungselement, welches die Vorrichtung optional aufweisen kann, dient dazu, das Schwingelement an den Schwingungsanreger zu koppeln. Es kann bspw. als Gewindezapfen ausgebildet und direkt auf den Schwingungsanreger aufschraubbar sein. Falls das Befestigungselement mit einem Scharnier und dem Schwingelement einstückig ausgebildet ist, weist die Vorrichtung unter Umständen nur ein einziges Bauteil auf, was auch für die Handhabung und unter herstellungstechnischen Gesichtspunkten vorteilhaft ist.

Gemäss einer weiteren Ausführungsform kann die Verbindung zwischen einem Befestigungselement und dem Schwingelement oder, falls die Vorrichtung kein Befestigungselement aufweist, direkt zwischen dem Schwingungsanreger und dem Schwingelement auch steif sein und bspw. als geschraubte, geklebte oder eventuell eingerastete, genietete oder sonstwie ausgebildete Verbindung vorhanden sein.

Falls die Vorrichtung ein Befestigungselement aufweist, befindet sich die Schwingungs-Aufnahmestelle meist an diesem. Das Befestigungselement kann dann mindestens bereichsweise zapfenförmig sein, wobei sich an der einen Stirnfläche des zapfenförmigen Bereichs die Schwingungs-Aufnahmestelle befindet, an die andere Stirnfläche ein Übergangsbereich zum Schwingelement anschliesst, welcher auch das Scharnier aufweist.

Obwohl eine feste (elastische oder steife) Verbindung zwischen dem Schwingungsanreger und der erfindungsgemässen Vorrichtung je nach Anwendung vorteilhaft sein kann, ist sie nicht notwendig. Vielmehr muss zwischen dem Schwingungsanreger und der erfindungsgemässen Vorrichtung gar keine stoffliche Verbindung vorhanden sein, sondern die Verbindung kann auch lose sein. Der Schwingungsanreger kann beispielsweise nur an die Vorrichtung angelegt werden, wobei dann der Schwingungsanreger nur durch Druckkräfte ("nur durch Schläge") auf die Vorrichtung einwirken kann und keine Zugkräfte ausüben kann - der Schwingungsanreger "hämmert" auf die Vorrichtung. Es besteht dann also nur während ungefähr einer halben Welle eine Kopplung zwischen Schwingungsanreger und Vorrichtung. In diesem Fall sind die Anregerfrequenz und die Eigenfrequenz der Vorrichtung mit Vorteil aufeinander abgestimmt, bspw. indem die Anregerfrequenz einer Eigenfrequenz oder einer harmonischen der Eigenfrequenz oder eines ganzzahligen Teilers der Eigenfrequenz der Vorrichtung entspricht.

Die Schwingungs-Abgabestelle fällt meist - aber nicht notwendigerweise - mit dem Auskoppelpunkt des Schwingelementes zusammen. An der Schwingungs-Abgabestelle sind geeignete Kupplungseinrichtungen vorgesehen, durch welche das Werkzeug, Werkstück oder Zwischenstück so mit der Vorrichtung temporär verbindbar ist, dass ein Benutzer des Gerätes während dem Beaufschlagen mit mechanischen Schwingungen des Werkzeugs, Werkstücks oder Zwischenstücks dasselbe auch im notwendigen Mass führen und insbesondere darauf einen Druck ausüben kann.

Eine Kupplungseinrichtung an der Schwingungs-Abgabestelle (also meist am Auskoppelpunkt) kann bspw. einen Kupplungszapfen an der Vorrichtung beinhalten, welcher in ein entsprechendes Loch im Werkzeug, Werkstück oder einem Zwischenstück eingreifen kann. Ein solcher Zapfen kann irgend eine geeignete Form aufweisen, bspw. eine zylindrische mit beliebiger Querschnittsform oder eine konische. Es können auch umgekehrt ein Loch in der Vorrichtung und ein entsprechender Zapfen im Werkzeug, Werkstück oder Zwischenstück vorhanden sein. Diese Kupplungseinrichtungen sind besonders einfach in der Herstellung und Handhabung. Je nach Situation ergibt sich aber die Gefahr von Verkantungen, und es können Winkelfehler erzeugt werden.

Alternativ zur Zapfen-Loch-Kupplung kann am Schwingelement bzw. am Werkzeug/Werkstück/Zwischenstück eine Kugelpfanne vorhanden sein, weiche mit einer entsprechenden Kugel des Gegenstücks zusammenwirkt. Dadurch werden Verkantungen vermieden. In Situationen, wo während der Bearbeitung die Orientierung des Werkzeugs/Werkstücks/Zwischenstücks durch das Schwingungen erzeugende Gerät definiert sein muss - und insbesondere Rotationen um eine longitudinale Achse verhindert werden sollten - kann eine Kugelpfanne verwendet werden, die verdrehsicher ist, indem sie nicht zylindersymmetrisch ist. Eine analoge Lösung ist auch für die Zapfen-Loch-Verbindung denkbar, bei welcher dann anstelle einer rotationszylindrischen Form eine im Querschnitt polygonale oder sonstwie nicht drehsymmetrische Form gewählt wird.

Auch an der Schwingungs-Abgabestelle ist eine feste elastische oder steife Verbindung zwischen der Vorrichtung und dem Werkzeug, Werkstück oder Zwischenstück nicht unbedingt notwendig. Vielmehr kann auch dort die Verbindung - geführt oder ungeführt - darin bestehen, dass die Vorrichtung an das Werkzeug, Werkstück oder Zwischenstück angelegt wird und in der Art eines Hammers wirkt. Im Falle einer festen Verbindung kann eine Kupplung zusätzlich Mittel beinhalten, mittels derer Zugkräfte an das Werkzeug, Werkstück oder Zwischenstück übertragbar sind. Solche separate Mittel können in an sich bekannter Art quer zur Vibrationsrichtung verlaufende, einander hintergreifende Elemente aufweisen, bspw. in der Art einer Bajonettverriegelung, eines Gewindes oder in einer anderen an sich bekannten Art.

Das Schwingelement kann bspw. aus Titan oder rostfreiem Stahl gefertigt sein. Alternativ dazu wird gemäss einer besonderen Ausführungsform ein Material für das Schwingelement verwendet, welches eine Dauerfestigkeit besitzt, d.h. dessen Wöhlerkurve für eine hohe Anzahl von Lastwechseln asymptotisch gegen einen von null verschiedenen Wert geht. Solche Materialien haben im Allgemeinen eine kubisch-raumzentrierte Struktur. Beispiele dafür sind ferritischer Stahl, bspw. Federstahl. Für Titan oder auch für Aluminium (ein anderes verwendbares Material) gilt das nicht. Trotzdem sind diese Materialien potentiell geeignet, wenn die Schwingungsauslenkungen des Schwingelementes im Betrieb klein sind im Vergleich zur maximalen möglichen Auslenkung. Andere mögliche Materialien sind Keramiken, metallische Gläser oder eventuell andere Gläser etc.

Gegenstand der Erfindung ist auch eine Einrichtung zum Umlenken von mechanischen Schwingungen beinhaltend eine erste und eine zweite Vorrichtung die je gemäss vorstehend ausgeführter Lehre ausgestaltet sind, wobei die Schwingungs-Abgabestelle der ersten Vorrichtung mit der Schwingungs-Aufnahmestelle der zweiten Vorrichtung gekoppelt ist, derart, dass eine transversale Schwingung der ersten Vorrichtung an deren Auskoppelpunkt eine transversale Schwingung der zweiten Vorrichtung an deren Einkoppelpunkt erzeugt. Durch diese Einrichtung kann der Bereich der zugänglichen Unlenkwinkel im Vergleich zur einzelnen Vorrichtung noch einmal erweitert werden. Ausserdem ergibt sich eine zusätzliche Variabilität. Insbesondere ist eine Umlenkung in zwei Ebenen möglich. Beispielsweise kann die zweite Vorrichtung relativ zur ersten Vorrichtung gesteuert und drehbar sein. Eine geeignete Dreheinrichtung ist dann bedienbar vom Handgriff her oder über eine geeignete Fernsteuerung, so dass der Benutzer nicht direkt an den - in manchen Situationen nicht zugänglichen - Vorrichtungen Hand anlegen muss. Alternativ dazu kann die Vorrichtung auch so ausgestaltet sein, dass die beiden Vorrichtungen in einem definierten Winkel zueinander fixiert sind. Dieser definierte Winkel kann bspw. ex situ anpassbar sein.

An der Schwingungs-Abgabestelle kann der zweiten Vorrichtung bspw. eine der vorstehend disktuierten Kupplungseinrichtungen vorhanden sein, durch welche ein Werkzeug, Werkstück (bspw. Implantat) oder ein Zwischenstück an die zweite Vorrichtung gekoppelt wird.

Ein Ultraschallgerät besitzt einen Schwingungsanreger, eine Schwingungsanreger-Ansteuerelektronik, und eine Vorrichtung (Vorrichtung), oder eine Einrichtung, die gemäss vorstehender Lehre ausgebildet ist.

Das Ultraschallgerät wird im Falle einer festen Kopplung zwischen dem Schwingungsanreger und der Vorrichtung (Sonotrode) vorzugsweise so betrieben, dass die Anregungsfrequenz unter der (ersten) Eigenfrequenz des Schwingelementes liegt. Eine von der Eigenfrequenz verschiedene Anregungsfrequenz ist darum vorteilhaft, weil dann die Ausgangsampliltude durch die Wahl der Eingangsamplitude gut kontrollierbar ist. Hingegen ist bei einem Betrieb in Resonanz die Amplitude des Schwingelementes (bzw. der Schwingung am Auskoppelpunkt) nur schwer kontrollierbar. Ausserdem müssen Schwingelement und Schwingungsanreger nicht durch eine Eichung aufeinander abgestimmt werden. Wenn die Anregungsfrequenz über der Eigenfrequenz läge, könnte eine Frequenzabsenkung aufgrund einer grossen Last während der Anwendung dazu führen, dass sich die Frequenz der Resonanzfrequenz nähert. Bei einer losen Kupplung zwischen Schwingungsanreger und Vorrichtung kann die Anregungsfrequenz der Resonanzfrequenz oder einem ganzzahligen Teiler dieser entsprechen.

Die Betriebsfrequenz des Ultraschallgerätes liegt vorzugsweise zwischen 15 kHz und 40 kHz. Bei einer Betriebsfrequenz von 20 kHz sollte die Resonanzfrequenz des Schwingelementes vorzugsweise mindestens 1 kHz, noch besser mindestens 2 kHz höher liegen als diese. Ganz allgemein wird das Gerät mit Vorteil mindestens 5% unter der Resonanzfrequenz des Schwingelementes betrieben.

Die Leistung des Ultraschallgerätes (d.h. die Nutzleistung des Schwingungsanregers) ist für die Implantation von Dentalimplantaten beispielsweise zwischen 20 W und 150 W, und die Amplitude (nach Umlenkung) der Schwingung liegt im Bereich zwischen 10 µm und 80µm, bspw. zwischen 20 µm und 80µm. Die benötigte Leistung hängt von der Masse des Werkzeugs/Werkstücks (ggf. inkl. eventuelle Zwischenstücke), der Dämpfung, von Reibungsverlusten etc. ab. Für die im Vergleich zu Dentalimplantaten relativ kleinen CMF-Implantate beträgt sie unter Umständen nur ca. 1/5 der obigen Werte. Für die Implantation von Implantaten für andere chirurgische Anwendungen kann die Leistung des Ultraschallgeräts und die Amplitude je nach Beschaffenheit des Implantates (als solches gilt auch ein Befestigungsmittel für ein grösseres Implantat, bspw. ein Stift zur Befestigung einer Gelenkpfanne) in einem grossen Bereich variieren. Die Leistungen hängen stark von der gewählten Applikation ab. Sie können bspw. zwischen 0.5 W und 300 W oder mehr - bspw. bis 2 kW -, die Amplituden zwischen 5 µm und 200µm liegen.

Die gewünschte Leistung bestimmt das Trägheitsmoment der Vorrichtung. Für diese ist die Steifigkeit des Schwingeleinentes ein massgeblicher Faktor, die ihrerseits von der Querschnittsfläche mit bestimmt wird. Bei einem Schwingelement aus Titan für die Implantation von Dentalimplantaten bei Frequenzen um 20 kHz kann die Querschnittsfläche zum Beispiel zwischen 10 und 50 mm² betragen.

Die erfindungsgemässe Vorrichtung und das erfindungsgemässe Ultraschallgerät sind einerseits besonders vorteilhaft bei einer Anwendung in der Zahnmedizin, der Cranio-Maxillo-Fazialen Chirurgie und der minimal-invasiven Chirurgie (MIS). Gemäss dem eingangs erwähnten neue entwickelten Verfahren zum Befestigen von Implantaten und Präparaten in porösem Material wird das Implantat bzw. Präparat in situ, d.h. am Patienten mit mechanischen Schwingungen beaufschlagt und ins verankernde Material getrieben. Die erfindungsgemässe Vorrichtung erlaubt die Umlenkung der mechanischen Schwingungen um jeden Winkel, insbesondere auch um beim intraoralen Zugang ergonomisch besonders günstige Winkel im Bereich von ungefähr 110° bis 120°, welche bspw. auch Zahnarztbohrgeräte als Winkel zwischen Griff und Bohrer vorsehen. Durch die erfindungsgemässe Form benötigt die Vorrichtung nur wenig Platz im Mund, die Arbeitshöhe ist gering. Dies ermöglicht die Arbeit auch unter schwierigen Bedingungen, bei Patienten, denen das weite Öffnen des Mundes Mühe bereitet und weit hinten im Mund. Auch für andere Anwendungen aus dem Cranio-Maxillo-Facial (CMF)-Chirurgie-Bereich ist die erfindungsgemässe Vorrichtung vorteilhaft. Auch für Anwendungen im Bereich der Mechanik, insbesondere Feinmechanik, bringt die Erfindung Vorteile.

Besonders bevorzugt weist die Vorrichtung noch eine Abdeckung in der Art eines Schutzgehäuses auf, welches unter anderem zur Verhinderung von Sekundärschäden dienen kann, indem es Körper- und Gewebeteile von der mechanisch schwingenden Vorrichtung abschirmt. Die Abdeckung kann gleichzeitig auch Führungsmittel zum Halten und Führen des Werkzeugs, Werkstücks oder Zwischenstücks aufweisen.

Für die Zwecke der minimalinvasiven Chirurgie kann vorgesehen sein, dass eine äussere Abmessung eines solchen Schutzgehäuses möglichst klein gehalten wird. Bspw. können die Vorrichtung und das sie umgebende Gehäuse so dimensioniert sein, dass sie in einer zylindrischen Röhre mit einem Innendurchmesser von maximal 8 mm Platz finden würden.

Ebenfalls besonders vorteilhaft sind die erfindungsgemässe Vorrichtung, die erfindungsgemässe Einrichtung und das erfindungsgemässe Ultraschallgerät andererseits für Anwendungen in der Implantationschirurgie, und der Knochenchirurgie Beispielsweise kann die als erfindungsgemässe Vorrichtung ausgestaltete Sonotrode auch bei chirurgischen Eingriffen im Zusammenhang mit Gelenkimplantaten und Wirbelsäulenimplantaten vorteilhaft sein. Sie eignet sich generell für das Befestigen von Implantaten an schwer zugänglichen Orten im menschlichen Körper, bspw. für das Befestigen von Bandscheibenimplantaten zwischen Wirbelknochen. Ebenfalls geeignet ist sie bspw. für das Fixieren von körpereigenen Gewebeteilen relativ zueinander.

Ebenfalls vorteilhaft kann eine Verwendung der erfindungsgemässen Vorrichtung, der erfindungsgemässen Einrichtung oder des erfindungsgemässen Ultarschallgeräts für Anwendungen sein, wie sie in der internationalen Offenlegungsschrift WO 2005/009 256 beschrieben sind.

Im Folgenden werden Ausführungsformen der Erfindung anhand von Zeichnungen im Detail erläutert. In den Zeichnungen zeigen:
- Fig. 1 eine schematische Darstellung eines Ultraschallgerätes mit einer - abgesetzt gezeichneten - Sonotrode sowie eines Implantat und eines Zwischenstücks,
- Fig. 2 eine Ansicht einer ersten Ausführungsform einer Sonotrode gemäss der Erfindung,
- Fig. 3a und 3b eine Ansichten von zwei Varianten einer zweiten Ausführungsform einer Sonotrode gemäss der Erfindung,
- Fig. 4 eine Ansicht einer dritten, mehrteiligen Ausführungsform einer Sonotrode gemäss der Erfindung,
- Fig. 5a bis 5j schematische Darstellungen von möglichen Formen des Schwingelementes,
- Fig. 6a bis 6d schematische Darstellungen von möglichen Kupplungsformen zum Kuppeln des Schwingelementes mit einem Werkzeug, Werkstück oder Zwischenstück,
- Fig. 7a bis 7e je eine schematische Darstellung einer Gelenkform zur Ankopplung des Schwingelementes an ein Befestigungselement,
- Fig. 8 eine Anordnung mit einer ersten und einer zweiten Sonotrode,
- Fig. 9 ein Beispiel der Kopplung der zwei Sonotroden, so, dass die zweite Sonotrode relativ zur ersten Sonotrode nicht drehbar ist,
- Fig. 10 ein Anwendungsbeispiel, nämlich die Fixierung einer Gelenkpfanne,
- Fig. 11 und 12 je ein weiteres Anwendungsbeispiel aus der Gelenkchirurgie, nämlich die Fixierung eines Tibia-Plateau-Implantats (künstlichen Schienbeinkopfs) auf zwei verschiedene Arten,
- Fig. 13 ein Anwendungsbeispiel aus der Wirbelsäulenchrirugie, nämlich die Fixierung einer-Bandscheibe an den Wirbelkörper,
- Fig. 14 ein weiteres Beispiel aus der Wirbelsäulenchrirugie, nämlich die Befestigung einer stabilisierenden Platte,
- Fig. 15 eine erfindungsgemässe Vorrichtung mit Schutzgehäuse, und
- Fig. 16 eine Ansicht einer weiteren Ausführungsform einer Sonotrode gemäss der Erfindung.

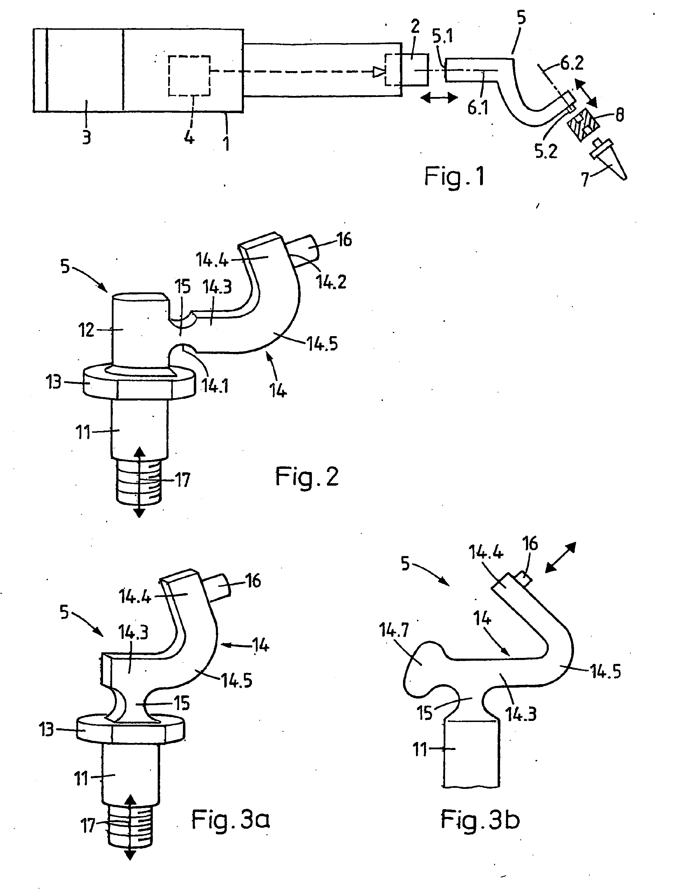

Das schematisch dargestellte Ultraschallgerät gemäss **Figur 1** ist geeignet zum Gebrauch als Handgerät. Es beinhaltet in an sich bekannter Art in einem Gehäuse 1 einen nicht dargestellten Piezo-Schwingquarz und Übertragungsmittel zum Übertragen einer Schwingung desselben auf einen Schwingungsanreger 2. Das Gehäuse hat eine längliche Grundform, was typisch für ist Instrumente für den dentalen Gebrauch. In der Zeichnung ist noch ein Handgriff 3 schematisch angedeutet. Ebenfalls schematisch dargestellt ist eine Ansteuer- und Anregungselektronik 4, welche eine Ausgangsspannung liefert, die den Piezo-Schwingquarz mit der gewünschten Frequenz und Amplitude in Schwingung versetzt.

Weiter sieht man in Figur 1 eine Sonotrode 5 die der Umlenkung der mechanischen Schwingungen dient, welche am Schwingungsanreger 2 abgegriffen werden. Wenn eine Schwingungs-Aufnahmestelle 5.1 der Sonotrode durch den Schwingungsanreger in Schwingungen längs einer ersten Achse 6.1 versetzt wird, erzeugt das eine Schwingung einer Schwingungs-Abgabestelle 5.2 längs einer zweiten Achse 6.2, die einen Winkel α (Umlenkwinkel) zur ersten Achse 6.1 bildet.

Ebenfalls - schematisch - dargestellt in der Figur 1 sind ein Dentalimplantat 7 und ein als Zwischen- bzw. Verbindungsstück dienendes Kunststoffelement 8 (bspw. aus PEEK, einem mechanisch und thermisch hochbelastbaren Kunststoff).

Je nach Verwendungszweck und Ausführungsform kann die Sonotrode um ihre erste Achse 6.1 drehbar sein, was bspw. dann sinnvoll ist, wenn das Ultraschallgerät nicht wie dargestellt im Wesentlichen zylindrisch ist, sondern eine andere, nicht zylindersymmetrische Form hat.

Ein erstes Beispiel für eine konkrete Ausgestaltung der erfindungsgemässen Sonotrode 5 zeigt **Figur 2****.** Das Befestigungselement beinhaltet einen Gewindezapfen 11. Dieser wird zu einem Übergangsbereich 12 hin durch einen Kragen 13 abgegrenzt. Das Schwingelement 14 ist an einem stirnseitigen Ende durch ein verbindendes Gelenk 15 an den Übergangsbereich 12 gekoppelt. Die Schwingungs-Abgabestelle fällt mit dem Auskoppelpunkt 14.2 zusammen, an welchem das Schwingelement einen Kupplungszapfen 16 aufweist, an den - eventuell via ein Zwischenstück - ein Werkstück (bspw. ein Implantat) oder ein Werkzeug angekoppelt werden kann.

Wenn die Sonotrode an der Schwingungs-Aufnahmestelle durch eine Schwingung in Richtung des Pfeils 17 angeregt wird - die Schwingung entspricht einer longitudinalen Schwingung des Gewindezapfens - wird über das Gelenk 15, welches an den Einkoppelpunkt 14.1 definiert, eine transversale Schwingung des Schwingelementes am Einkoppelpunkt angeregt. Es entsteht eine (Grund-) Schwingung, bei welcher der erste Arm 14.3 und der zweite Arm 14.4 des V-förmigen Schwingelementes in der Art einer Stimmgabel gegeneinander schwingen. Aufgrund der Wirkung des scharnierartigen Gelenks 15 geht diese Schwingung nicht mit einer entsprechenden Schwingung des Schwingelement-Massenschwerpunktes einher, sondern vielmehr kann der Erste Arm vom Übergangsbereich nach oben oder unten "wegkippen" so dass ein Neutralpunkt 14.5 bei der Schwingung ungefähr stationär bleibt. Der Winkel zwischen der Longitudinalrichtung des Schwingelementes am Einkoppelpunkt 14.1 und der Longitudinalrichtung am Auskoppelpunkt beträgt 110°, d.h. der Winkel zwischen den beiden Armen 14.3, 14.4 ungefähr 70°.

Die Querschnittsform (in einem Querschnitt senkrecht zur Longitudinalrichtung) des Schwingelementes ist ungefähr rechteckig und so, dass das Element in Richtung senkrecht zur Zeichnungsebene eine grössere Breite aufweist als in der anderen Richtung. Das hilft, Schwingungen senkrecht zur Schwingelement-Ebene zu unterdrücken.

Die Ausführungsform der Sonotrode gemäss **Figur 3a** unterscheidet sich von derjenigen von Figur 2 dadurch, dass das Gelenk 15 nicht stirnseitig, sondern lateral an das Schwingelement anschliesst. Dadurch wird eine kompaktere Bauweise möglich, da der Übergangsbereich der Sonotrode gemäss Figur 2 entfallen kann. Das Gelenk 15 wird aufgrund einer longitudinalen Schwingung (in Richtung des Pfeils 17) des Befestigungselementes 11 quasi gestossen. Es hat aber eine ähnliche Wirkung wie in Figur 2, d.h. es ermöglicht ein Wegkippen des Schwingelements, wodurch der Neutralpunkt 14.5 bei der Schwingung ungefähr stationär bleiben kann.

Die - nur schematisch dargestellte - Variante von **Figur 3b** unterscheidet sich von derjenigen von Figur 3a dadurch, dass im Bereich des ersten Arms 14.3 eine zusätzliche Masse 14.7 vorhanden ist. Durch diese können die Resonanzeigenschaften der Vorrichtung und damit das Schwingungsverhalten an der Schwingungs-Abgabestelle und die Belastungen im Bereich des Scharniers 15 und des Neutralpunkts 14.5 beeinflusst und optimiert werden. Die Vorrichtung gemäss Figur 3b weist demnach auf:
- ein im Betrieb longitudinal schwingendes Befestigungselement 11, das bspw. als Gewindezapfen ausgebildet sein kann,
- ein in longitudinaler Richtung daran anschliessendes Scharnier 15,
- ein am Scharnier befestigtes Schwingelement 14 mit einem ersten Arm 14.3 und einem zweiten Arm 14.4, wobei das Scharnier 15 am ersten Arm angebracht ist, und wobei die beiden Arme gerade oder gebogen sein können,
- einen zwischen dem ersten Arm und dem zweiten Arm befindlichen gebogenen bzw. stärker gebogenen Bereich,
- wobei ein Übergang zwischen dem Scharnier 15 und dem ersten Arm 14.3 bezüglich des ersten Arms seitlich ist,
- wobei auf einer bezüglich dem genannten Übergang dem gebogenen Bereich gegenüberliegenden Seite des ersten Arms 14.3 eine Gegenmasse am ersten Arm vorhanden ist (d.h. der erste Arm reicht "nach hinten" über den Übergang hinaus),
- und wobei das Befestigungselement, das Scharnier und das Schwingelement einstückig sind.

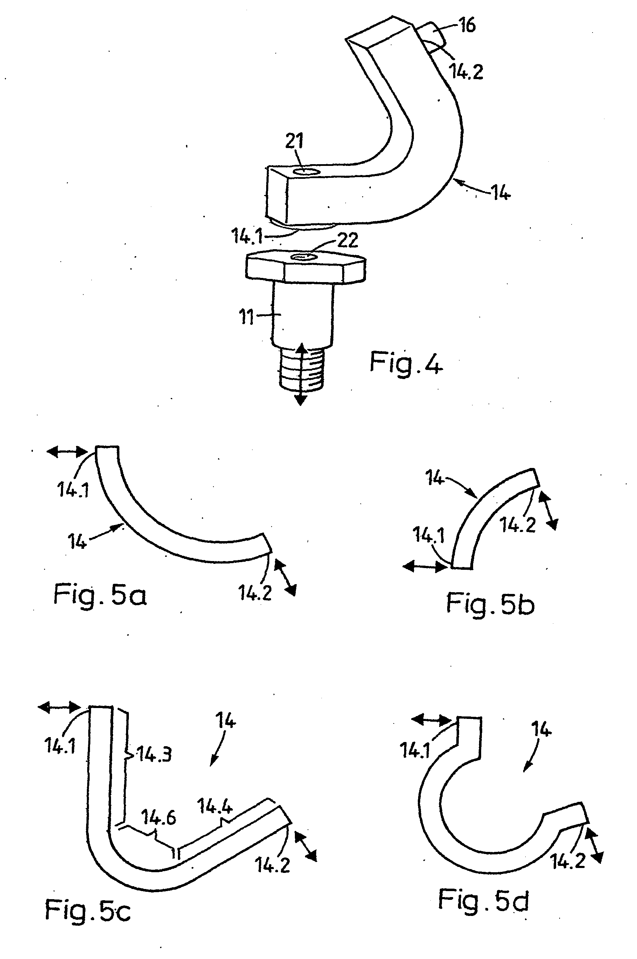

Die Ausführungsform gemäss **Figur 4** ist ähnlich derjenigen von Figuren 3a und 3b, unterscheidet sich aber von dieser dadurch, dass die Sonotrode 5 nicht einstückig ist, sondern das Befestigungselement 11 vom Schwingelement separat ist und durch ein Verbindungselment, beispielsweise eine Schraube (nicht gezeichnet) an dieses koppelbar ist. In der Figur sichtbar sind ein Durchgangsloch 21 für die Schraube im Schwingelement und ein entsprechendes Sackloch 22 im Befestigungselement 11. Je nach Elastizität des Schraubenmaterials kann die geschraubte Verbindung im Wesentlichen fest sein oder sie kann ebenfalls als Gelenk wirken, wie das weiter unten noch dargelegt wird.

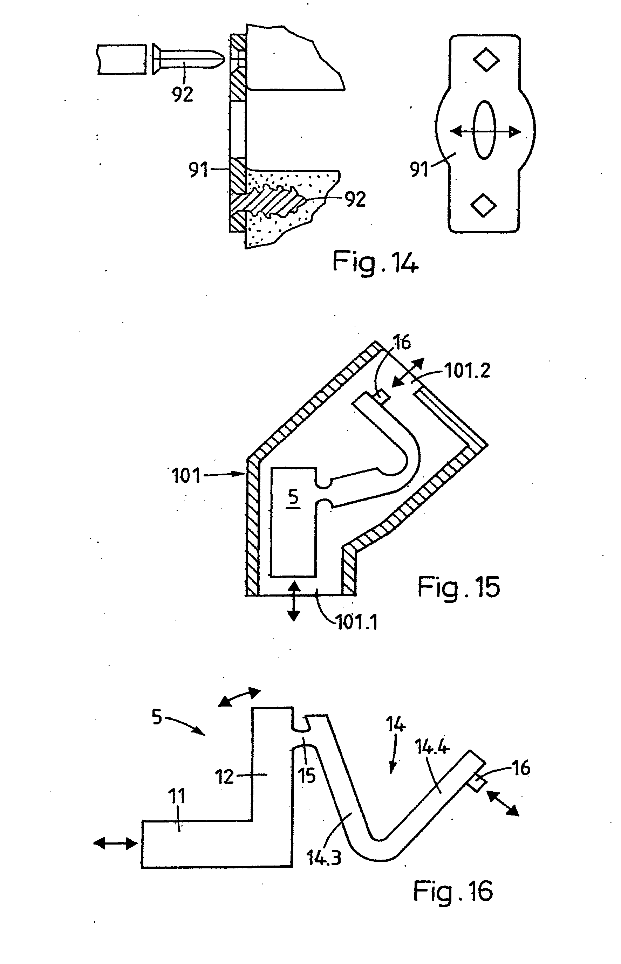

Während in den Ausführungsformen der Figuren 2-4 die Schwingungsanregung "in axis" erfolgt - d.h. die Elemente zwischen dem Schwingungsanreger 2 und dem Schwingelement 14 liegen alle auf einer gemeinsamen Achse - sind auch alternative Möglichkeiten denkbar. Eine solche alternative Möglichkeit ist sehr schematisch in **Figur 16** illustriert. Das Befestigungselement hat eine L-Form, so dass der Ansatzpunkt des Schwingelements 14 - gebildet druch das Scharnier 15 - von der Achse versetzt angeordnet ist. Nebst der unterschiedlichen Geometrie - diese kann je nach Anwendung vorteilhaft sein - hat das zur Folge, dass der Übergangsbereich 12 relativ zum Gewindezapfen 11 in Kipp-Schwingungen versetzt werden kann. Diese können einen Einfluss auf Resonanzen des Systems und auf die Amplitude an der Schwingungs-Abgabestelle haben.

In den Figuren 5a bis 5i sind sehr schematisch mögliche Formen von Schwingelementen einer erfindungsgemässen Sonotrode gezeichnet. Die Schwingelemente können - ggf. via Gelenke - an beliebigen Befestigungselementen oder direkt am Schwingungsanreger befestigbar sein oder einstückig mit den Befestigungselementen ausgeformt sein. Sie können verschiedene Kupplungseinrichtungen aufweisen. Bei allen Schwingelementen wird von einem Umlenkwinkel von ungefähr 100°-120° ausgegangen; entsprechende Modifikationen für andere Winkel sind selbstverständlich möglich und offensichtlich.

Das Schwingelement 14 gemäss **Figur 5a** ist bogenförmig, d.h. über seine ganze Länge gekrümmt, wobei der Krümmungswinkel konstant sein kann, aber nicht konstant sein muss.

Auch des Schwingelement gemäss **Figur 5b** ist bogenförmig. Es unterscheidet sich von demjenigen gemäss Figur 5a dadurch, dass der Auskoppelpunkt 14.2 an der Innenseite liegt, weshalb der Biegewinkel 180° minus dem Umlenkwinkel beträgt. Das Schwingelement ist zum Werkstück, Werkzeug oder Zwischenstück hin gekrümmt.

Bei den im Folgenden gezeichneten Varianten des Schwingelementes ist der Auskoppelpunkt jeweils auf der Aussenseite; Modifikationen für den Fall eines innenseitigen Auskoppelpunktes sind aber in jedem der gezeichneten Fälle möglich. Falls der Umlenkwinkel im Bereich zwischen 190° und 130° liegen soll, geht eine solche Modifikation mit einer Änderung des Biegewinkels auf zwischen 50° und 80° einher. Insgesamt ergibt sich ein im Allgemeinen interessierender Bereich von Biegewinkeln zwischen 50° und 130°.

Das Schwingelement gemäss **Figur 5c** ist V-förmig, d.h. an den gebogenen Abschnitt 14.6 schliessen zwei Arme 14.3, 14.4 an. Die beiden Arme können, müssen aber nicht dieselben Abmessungen (Länge, Querschnittsfläche bzw. Querschnittsflächenverlauf) und dieselbe Ausgestaltung (Querschnittsform, Oberflächenbeschaffenheit etc.) haben.

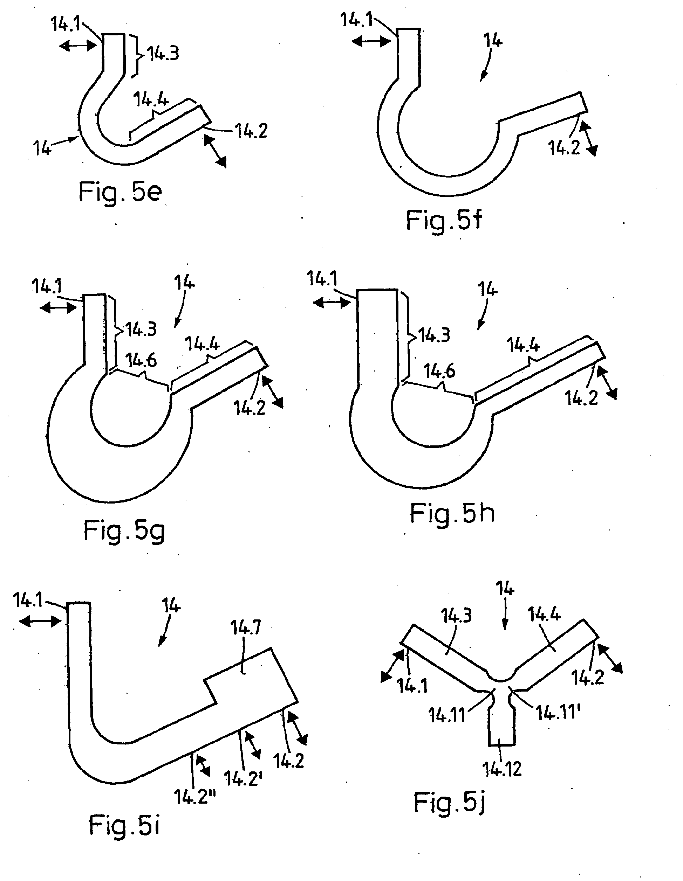

**Figur 5d** zeigt ein Omega-förmiges Schwingelement, während **Figur 5e** ein hakenförmiges Schwingelement darstellt. Das hakenförmige Schwingelement kann als Kombination der Konzepte eines Omega-förmigen Schingelementes (erster Arm 14.3) mit einem V-förmigen Schwingelement (zweiter Arm 14.4) betrachtet werden. Ganz generell sind solche Kombinationen möglich, also auch bogenförmig-V-förmig, bogenförmig-Omega-förmig etc.

In **Figur 5f** sieht man ein Schwingelement, welches quasi eine Zwischenlösung zwischen einem Omega-förmigen und einem V-förmigen Schwingelement betrachtet werden kann. Ein solches Schwingelement wirkt ähnlich einem V-förmigen schwingelement, es ist aber spannungsoptimiert.

**Figur 5g** zeigt eine Variante des Schwingelements von Figur 5f, wobei die Dicke (also die Querschnittsfläche) im gebogenen Abschnitt 14.6 im Vergleich zu den Armen 14.3, 14.4 vergrössert ist. Ein solches Schwingelement ist etwas steifer als dasjenige von Figur 5f, hat also eine höhere Eigenfrequenz bei gegebenen Materialeigenschaften.

Das Schwingelement von **Figur 5h** ist ebenfalls eine Variante desjenigen von Figur 5f, wobei die beiden Arme 14.3, 14.4 unterschiedliche Dicken und folglich auch unterschiedliche Massen haben. Wenn wie gezeichnet der Arm 14.3 mit dem Einkoppelpunkt die grössere Masse hat, bewirkt das Schwingelement eine Amplitudenverstärkung.

Umgekehrt besitzt das Schwingelement von **Figur 5i**, eine Variante des Schwingelementes von Fig. 5c, eine Extramasse 14.7 im Bereich des Auskoppelpunktes, was eine Amplitudenuntersetzung zur Folge hat.

Das Schwingelement 14 von **Fig. 5j** weist zwischen einem Mittelteil 14.12 und den beiden Armen 14.3, 14.4 je eine scharnierartige Verengung 14.11, 14.2 auf. Die Verengungen verringern die Steifigkeit des Schwingelements als Ganzes und setzen daher bei gleicher Grösse im Vergleich zu einem Schwingelement wie in Fig. 5c die Resonanzfrequenz und damit, je nach Betriebsparametern, auch die optimale Arbeitsfrequenz herunter. Es ist auch möglich, ein Schwingelement gemäss Fig. 5j vergleichsweise kleiner zu dimensionieren. Dadurch kann man das Schwingelement so auslegen, dass bei gleichen Materialien die Resonanzfrequenz in einem ähnlichen Bereich ist wie bei einem grösseren Schwingelement gemäss Fig. 5c. Eine reduzierte Steifigkeit des Schwingelements ist also ein Weg, das Schwingelement und mithin die ganze Vorrichtung zu miniaturisieren.

Die Varianten der Figuren 5g-5i sind auch auf die Schwingelemente der Figuren 5a bis 5e bzw. 5a, 5b und 5d bis 5f anwendbar; auch Kombinationen der Varianten untereinander sind möglich.

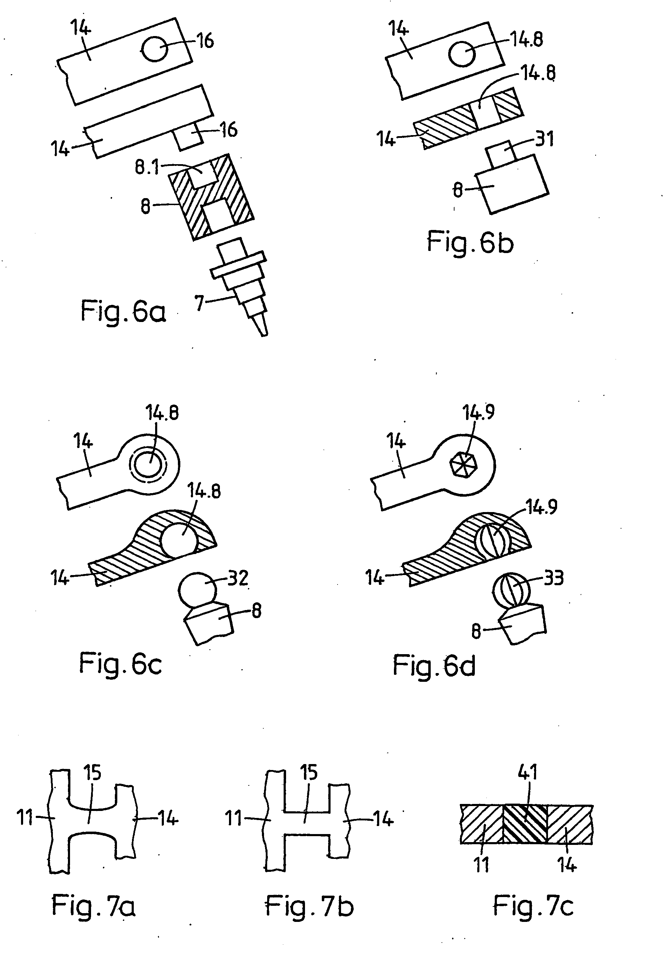

Figuren 6a bis 6d zeigen Beispiele von Kupplungselementen. In den Figuren ist jeweils ein Auskoppelpunkt-seitiger Arm eines Schwingelementes 14 in zwei verschiedenen Ansichten bzw. Schnittdarstellungen gezeichnet.

Die Kupplung gemäss **Figur 6a** wird durch das Zusammenspiel eines Kupplungszapfens 16 mit einer entsprechenden Aussparung 8.1 im Verbindungsstück 8 bewirkt. Die umgekehrte Anordnung (Loch 14.8 im Schwingelement, Zapfen 31 am Verbindungsstück) zeigt **Figur 6b**. Diese Art der Kupplung ist einfach in der Herstellung und ermöglicht ein einfaches Ankoppeln, sie ist aber anfällig auf Winkelfehler und nicht rotationssicher. Wenn anstelle eines kreiszylindrischen Zapfens ein anderer, nicht drehsymmetrischer Zapfen verwendet wird - es kann ein polygonaler, elliptischer, sternförmiger etc. Querschnitt gewählt werden - ist eine zu Fig. 6a bzw. Fig. 6b analoge Kupplung rotationssymmetrisch.

Bezüglich der Anfälligkeit auf Winkelfehler schafft sie Kugelpfannen-Kupplung gemäss **Figur 6c** Abhilfe: Ein Kugelelement 32 des Verbindungsstücks 8 wirkt mit einer gelenkpfannenartigen Aussparung 14.8 des Schwingelementes zusammen. Diese Verbindungsart ist ebenfalls nicht verdrehsicher. Es ist aber auch eine verdrehsichere Variante möglich, wie **Figur 6d** zeigt. Gemäss dieser Ausführungsform besitzt das Verbindungsstück 8 ein nicht zylindersymmetrisches Kugelelement 33, welches mit einer entsprechenden Vertiefung 14.9 des Schwingelementes zusammenwirkt. Auch die Varianten gemäss Figuren 6c und 6d können selbstverständlich in der umgekehrten Anordnung ausgestaltet sein. Weitere Kupplungsvarianten sind denkbar, bspw. mit leicht konischen Zapfen etc.

Zusätzlich zu den gezeichneten Elementen kann eine Kupplung auch separate Mittel beinhalten, mittels derer Zugkräfte an das Werkzeug, Werkstück oder Zwischenstück übertragbar sind. Dies ist unter Umständen notwendig bei Ausführungsformen, bei denen eine feste Verbindung zwischen der Vorrichtung und dem Werkzeug, Werkstück oder Zwischenstück gewünscht wird und bei denen die Reibungs- und/oder Klemmkräfte obiger Kupplungsmittel nicht ausreichen. Solche separate Mittel können in an sich bekannter Art quer zur Vibrationsrichtung verlaufende, einander hintergreifende Elemente aufweisen, bspw. in der Art einer Bajonettverriegelung oder in einer anderen an sich bekannten Art.

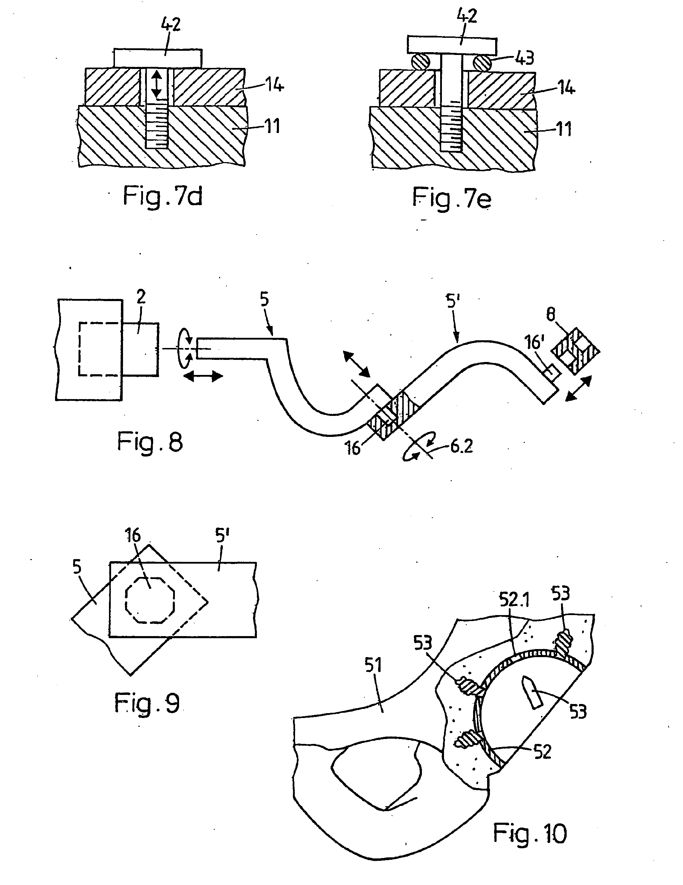

In den Figuren 7a bis 7e sind sehr schematisch verschiedene Ausführungsformen von Verbindungen zwischen einem Befestigungselement 11 (oder, als Alternative für nicht einstückige Ausführungsformen, dem Schwingungsanreger) und dem Schwingelement 14 gezeichnet. Das Gelenk 15 gemäss **Figur 7a** entspricht dem bereits vorstehend erörterten. **Figur 7b** zeigt eine Variante davon. Gemäss **Figur 7c** wird das Gelenk durch eine Inhomogenität des Materials beim Übergang zwischen Befestigungselement 11 und Schwingelement 14 gebildet: Im Übergangsbereich 41 besteht die Sonotrode aus einem Material mit einem im Vergleich zum Schwing- und Befestigungselement kleineren Elasitizitätsmodul. Gemäss **Figur 7d** ist die Verbindung eine Schraubverbindung (wie in Figur 4) wobei die Schraube 42 dehnbar ist weil sie einen vergleichsweise geringen Durchmesser aufweist und/oder aus einem Material mit einem kleineren Elastizitätsmodul gefertigt ist. Anstatt als Schraubverbindung kann die Verbindung auch auf andere vergleichbare Art erfolgen bspw. als Bajonettverbindung. **Figur 7e** schliesslich zeigt eine Variante einer Schraubverbindung mit einer nicht oder nicht wesentlich dehnbaren Schraube und einem kompressiblen Federelement 43, das bspw. an der Schraube befestigt sein kann. Auch hier ist anstelle einer Schraube ein anderes Verbindungsmittel denkbar.

Varianten mit anderen Geometrien, Befestigungsmitteln etc. sind selbstverständlich möglich.

In diesem Text wird wie vorstehend mehrfach erwähnt mit "Schwingelement" der Teil der Vorrichtung bezeichnet, der als ganzes eine längliche Form hat, gebogen ist und die eigentliche Schwingung ausführt - analog zu den zwei Armen einer Stimmgabel. Der Auskoppelpunkt ist diejenige Stelle, an der die Schwingung vom Schwingelement abgegriffen wird. In allen gezeichneten Ausführungsformen fällt die Schwingungs-Abgabestelle mit dem Auskoppelpunkt zusammen. Das ist nicht eine notwendige Voraussetzung. Abweichend davon kann die Sonotrode beispielsweise zwischen dem Auskoppelpunkt und der Schwingungs-Abgabestelle ein Übergangselement aufweisen, das irgend eine geometrische Form hat und durch das die Schwingungen bspw. als Longitudinalschwingungen vom Auskoppelpunkt an die Schwingungs-Abgabestelle übertragen werden. Die Kupplungseinrichtung ist dann im Allgemeinen am vom Schwingelement entfernten Ende des Übergangselementes vorhanden. Ein solches Übergangselement kann bspw. die Form eines fest mit dem Schwingelement verbundenen Stäbchens haben und ein Ansetzen des Gerätes an versenkten Orten erlauben.

In allen vorstehend diskutierten Beispielen befinden sich der Einkoppelpunkt und der Auskoppelpunkt jeweils in der Nähe der Enden des Schwingelements. Dies ist keine Notwendigkeit. Vielmehr kann bspw. durch die Wahl des Auskoppelpunktes die Amplitude der ausgekoppelten Schwingung beeinflusst werden. Dies wird exemplarisch in **Fig. 5i** durch Doppelpfeile skizziert. Bei einer Verschiebung des Auskoppelpunktes vom äusseren Ende 14.2 gegen innen an alternative Auskoppelpunkte 14.2', 14.2" kann die Amplitude im Prinzip stufenlos gesenkt werden. Je weiter weg vom in der Figur rechten Ende des Schwingelements der Auskoppelpunkt angesetzt ist, desto kleiner ist die Amplitude der ausgekoppelten Schwingung. Analog kann natürlich auch der Einkoppelpunkt entlang des ersten Armes verschoben werden. Ausserdem kann distal vom Auskoppelpunkt ein Gegengewicht vorhanden sein, mit dem Amplitude und Resonanzfrequenz beeinflusst werden können. (Wird in Fig. 5i der Auskoppelpunkt an der Stelle 14.2'' angesetzt, wirkt die Extramasse als solches Gegengewicht). Alternativ oder ergänzend kann ein solches Gegengewicht auch distal vom Einkoppelpunkt zur Verfügung gestellt werden.

Diese Überlegungen betreffend Ein- und Auskoppelpunkt sowie Gegengewichten wurden exemplarisch anhand des Ausführungsbeispiels gemäss Fig. 5i diskutiert. Sie gelten analog für alle anderen Schwingelement-Geometrien.

In **Figur 8** ist schematisch eine Anordnung mit zwei Vorrichtungen (Sonotroden) der erfindungsgemässen Art gezeichnet, die zusammen eine Einrichtung zum Umlenken von mechanischen Schwingungen bilden. Die erste Sonotrode 5 ist nicht in direktem Kontakt mit dem Werkstück oder Verbindungsstück 8 sondern dient als Übergangsstück (Konverter), welches die am Schwingungsanreger 2 abgenommenen Schwingungen um einen ersten Umlenkwinkel umlenkt und in die zweite Sonotrode 5' einkoppelt. Diese ist ebenfalls erfindungsgemäss ausgestaltet und lenkt also die Schwingungen um einen zweiten Unlenkwinkel um. Der erste Unlenkwinkel kann dem Betrag nach gleich gross sein wie der erste Umlenkwinkel, oder die beiden Umlenkwinkel können verschieden sein.

Die Verbindung zwischen der ersten und der zweiten Sonotrode kann gemäss einer ersten Alternative fest sein. Die Orientierung der zweiten Sonotrode relativ zur ersten kann aber auch beeinflussbar sein. Beispielsweise kann die Orientierung vor der Operation - ex situ - einstellbar und bspw. mit einer Feststellschraube fixierbar sein.

Gemäss einer weiteren Alternative kann die Verbindung beweglich und die relative Orientierung der Sonotroden in situ veränderbar sein. So kann die zweite Sonotrode um die zweite Achse 6.2 der ersten Sonotrode drehbar sein. Dies kann bspw. durch eine vom Ultraschallgerät her bedienbare Dreheinrichtung geschehen. Eine solche kann in an sich bekannter Art Kraft- und/oder Drehmomentumlenkeinrichtungen (Seilzüge etc.) aufweisen, die bspw. von einem elektrischen Antrieb oder eventuell von Hand in Betrieb gesetzt werden. Alternativ dazu können Antriebsmittel der Dreheinrichtung auch direkt am Ort der Kupplung zwischen der ersten und der zweiten Sonotrode angreifen. Beispielsweise kann je nach Ausgestaltung der Sonotrode durch mechanischen Schwingungen in einem Frequenzbereich, welcher von der Arbeitsfrequenz verschieden ist, in der ersten Sonotrode eine Schwingung ausgelöst werden, welche eine Drehung der zweiten Sonotrode bewirkt, ähnlich dem Prinzip eines Piezomotors.

In **Figur 9** ist eine alternative Ausführungsform einer Kupplung gezeichnet, in welcher ein Kupplungszapfen 16 der einen Sonotrode und die entsprechende Aussparung der anderen Sonotrode nicht drehsymmetrisch sind, sondern bspw. im Querschnitt Vieleck-förmig. Eine derartige Kupplung erlaubt das Anordnen der beiden Sonotroden relativ zueinander in einer diskreten Anzahl von definierten Orientierungen. In einer einmal gewählten Orientierung sind die beiden Sonotroden fixiert, eine Anpassung der Orientierung ist nur ex situ durch Herausnehmen des Kupplungszapfens 16 der einen Sonotrode aus der entsprechenden Aussparung der anderen Sonotrode und wiedereinführen in einer anderen Orientierung möglich.

**Figur 10** zeigt ein Verfahren, bei welchem die erfindungsgemässe Sonotrode und auch die Vorrichtung zum Umlenken von mechanischen Schwingungen verwendbar sind. Sehr schematisch und ausschnittweise gezeichnet ist ein Beckenknochen 51, in dem eine künstliche Hüftgelenkpfanne 52 eingesetzt wird. Dies geschieht mit einer Mehrzahl von Implantaten 53, die gemäss dem in den Schriften WO 02/069'817 und WO 2004/017 857 beschriebenen Prinzip ausgestaltet sind.

In einem ersten Schritt wird die Hüftgelenkpfanne gemäss an sich bekannten Verfahren plaziert. Anschliessend werden durch dafür vorgesehene Öffnungen 52.1 Implantate der genannten Art geführt und durch Verflüssigung von thermoplastischem oder thixotropen Material an seiner Oberfläche mittels Applikation von mechanischen Schwingungen im porösen Knochenmaterial verankert. Optional können vor der Applikation der mechanischen Schwingungen Bohrungen im Knochen erfolgen, damit das Einbringen der Implantate mit wenig Kraftaufwand geschehen kann. Die Implantate können so ausgebildet sein, dass sich beim Beaufschlagen mit mechanischen Schwingungen auf der proximalen Seite eine Art Kopf bildet, durch den die künstliche Hüftgelenkpfanne fixiert wird. Alternativ dazu kann auch ein vorgefertigter Kopf vorhanden sein, und/oder es kann zu einer Infiltration von thermoplastischem Material des Implantats in einen porösen Oberflächenabschnitt der Hüftgelenkpfanne kommen. Als weitere Alternative kann die Hüftgelenkpfanne Bereiche aus Kunststoff aufweisen, und es kommt zu einer Verschweissung dieser Bereiche mit Bereichen des Implantats.

Beim Verfahren gemäss Figur 10 kommen die Vorteile der Erfindung besonders gut zur Geltung: die verschiedenen Implantate müssen unter unterschiedlichen Winkeln in den knochen eingetrieben werden. Durch die Umlenkung der mechanischen Schwingungen mit einer erfindungsgemässen Sonotrode ist das sehr einfach machbar. Besonders vorteilhaft ist die Verwendung einer Vorrichtung gemäss Figur 9, insbesondere wenn die zweite Sonotrode durch den Benutzer in situ - also ohne dass das Gerät von der Operationsstelle entfernt werden müsste - durch eine Dreheinrichtung relativ zur ersten gedreht werden kann.

Auch wenn in Figur 10 das Beispiel einer Hüftgelenkpfanne gezeichnet ist, ist ein analoges Vorgehen auch für andere vergleichbare Operationen denkbar, bspw. für ein Schultergelenk.

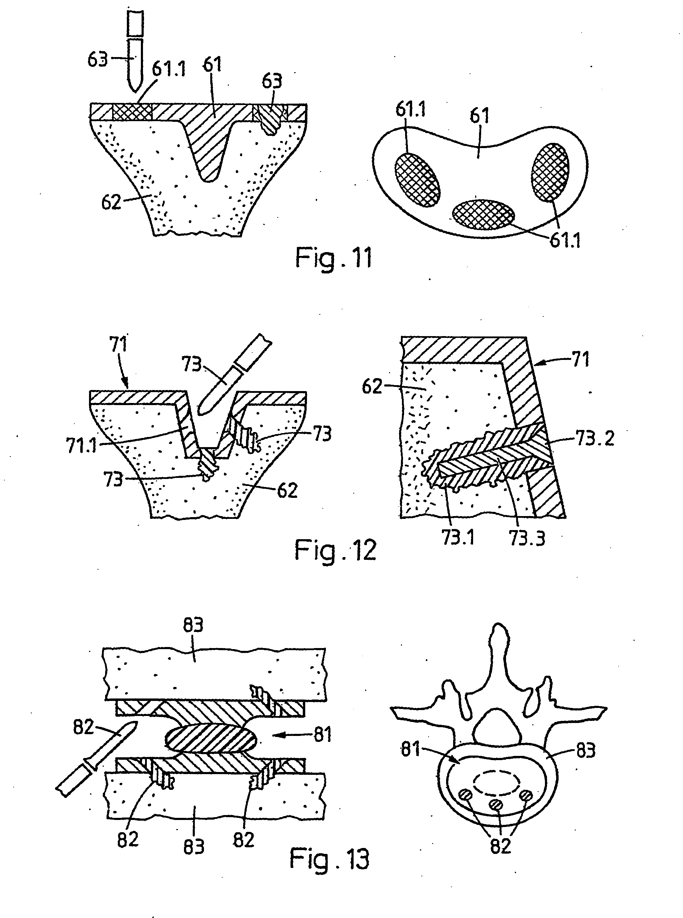

**Figur 11** zeigt die Fixierung eines Tibia-Plateau-Implantats 61 gemäss einer ersten Variante. Das Tibia-Plateau-Implantat 61 weist vordefinierte offenporig poröse Zonen 61.1 auf. Er wird in einem ersten Schritt mittels an sich bekannten Verfahren in seine endgültige Position am Schienbeinknochen 62 gebracht. Anschliessend wird ein (thermoplastisches oder eventuell thixotropes) Polymer durch die porösen Zonen 61.1 eingebracht, was durch Beaufschlagen eines als Polymerkörpers ausgebildeten Präparats 63 mit mechanischen Schwingungen und gleichzeitigem Anpressen gegen die porösen Zonen geschieht. Das Polymer infiltriert nebst den porösen Zonen auch den Knochen und sorgt so für eine Primärstabilität. Die porösen Zonen sind in der Folge vorteilhaft bei der Osseointegration und ermöglichen, dass das Tibia-Plateau-Implantat mit dem Knochen gut verwächst.

Ein zu Figur 11 analoges Vorgehen - Einbringen eines Polymer-Präparates durch poröse Zonen - kann auch zur Anwendung kommen, wenn eine Gelenkpfanne am Knochen befestigt werden kann.

Die zweite Variante zur Fixierung eines Tibia-Plateau-Implantats 71 am Schienbeinknochen 62, wie in **Figur 12** gezeigt, beruht auf dem Verfahren gemäss den Schriften WO 02/069'817 und WO 2004/017 857. In der Figur links ist ein Schienbeinknochen 62 mit dem Tibia-Plateau-Implantat 71 vor der Implantierung eines zweiten Implantats 73 gezeichnet, rechts sieht man einen Ausschnitt mit implantiertem Implantat. Das Tibia-Plateau-Implantat wird zuerst mittels an sich bekannten Verfahren, welche auch die operative Vorbereitung des Schienbeinknochens beinhalten, in seine endgültige Position gebracht. Anschliessend werden Implantate 73, deren Oberflächen mindestens teilweise ein theromplastisches oder thixotropes Polymer 73.1 aufweisen, durch vorgefertigte Öffnungen 71.1 des Tibia-Plateau-Implantats in den Knochen eingetrieben. Auch hier können optional vorgängig Vorbohrungen im Knochen erfolgen. Wie beim Verfahren gemäss Figur 10 können die Implantate 73 so ausgebildet sein, dass sich beim Beaufschlagen mit mechanischen Schwingungen auf der proximalen Seite eine Art Kopf bildet, durch den der künstliche Schienbeinkopf fixiert wird. Alternativ dazu kann auch ein vorgefertigter Kopf 73.2 vorhanden sein, und/oder es kann zu einer Infiltration von thermoplastischem Material des Implantats in einen porösen Oberflächenabschnitt des künstlichen Schienbeinkopfs kommen, oder das Implantat kann bereichsweise letzterem verschweisst werden. In der gezeichneten Ausführungsform besitzt das Implantat einen harten Kern 73.3, bspw. aus Titan oder einem anderen geeigneten nicht verformbaren Material.

Der Schienbeinkopf ist während einer Operation wie denen gemäss Figuren 11 und 12 von der Seite her zugänglich, mechanischen Schwingungen und der Druck müssen aber von oben oder schräg oben her angewandt werden. Aus diesem Grund ist die Verwendung einer erfindungsgemässen, mechanische Schwingungen um einen Umlenkwinkel umlenkenden Sonotrode hier besonders vorteilhaft. Auch eine Vorrichtung gemäss Figur 9 kann verwendet werden.

In den **Figuren 13** **und** **14** werden noch Verfahren aus der Wirbelsäulenchirurgie gezeigt, welche mit dem erfindungsgemässen Vorgehen realisierbar sind.

**Figur 13** zeigt eine die Fixierung einer künstlichen Bandscheibe (d.h. eines Bandscheibenimplantats) an den Wirbelkörpem der beiden benachbarten Wirbelknochen. Als Bandscheibenimplantat 81 kann ein Standard-Bandscheibenimplantat verwendet werden. Dieses wird zuerst mit an sich bekannten Verfahren zwischen den Wirbelkörpern plaziert. Anschliessen werden Implantate 82 mit dem Verfahren gemäss den Schriften WO 02/069'817 und WO 2004/017 857 eingeführt, um das Implantat zu befestigen. Dabei infiltriert Polymermaterial, das mittels Beaufschlagen mit mechanischen Schwingungen verflüssigt wird, poröses Material des Wirbelknochens 83 und gemäss einer ersten Variante auch poröses Material des Bandscheibenimplantates 81. Gemäss einer zweiten Variante verschmilzt das Polymermaterial des Implantates 82 oberflächlich mit Bandscheibenimplantat-Material in der Art einer Ultraschallschweissung. Gemäss weiteren Varianten erfolgt die Fixierung des Bandscheibenimplantates wie in den vorstehend beschriebenen Ausführungsbeispielen erwähnt mittels eines (versenkten) Kopfs, der vorgeformt ist oder sich beim Einbringen bildet.

Auch beim Verfahren gemäss Figur 13 ist die Umlenkung mechanischer Schwingungen durch die erfindungsgemässe Vorrichtung und ggf. durch die erfindungsgemässe Einrichtung von grossem Vorteil, da die Implantate 82 an sehr schwer zugänglicher Stelle und in einem Winkel in den Knochen eingetrieben werden.

**Figur 14** schliesslich zeigt eine Platte 91, die an zwei Wirbelknochen befestigt und zur Stabilisierung der Wirbelsäule verwendet werden kann. Die Platte ist mindestens bereichsweise aus elastischem Material gefertigt und kann wie durch den Doppelpfeil symbolisiert zusammengedrückt und gedehnt werden, wobei sich der mittlere Teil deformiert. Sie wird vorzugsweise durch im Querschnitt nicht kreisförmige Implantate 92 befestigt, wodurch sich eine vollständige mechanische Stabilität ergibt auch wenn insgesamt nur zwei Implantate 92 zur Befestigung verwendet werden. Für die Befestigung der Implantate im Knochenmaterial und der Platte 91 an den Implantaten wird das in den Schriften WO 02/069'817 und WO 2004/017 857 gezeigte und anhand der vorstehenden Ausführungsbeispielen diskutierte Verfahren verwendet.

Die in den vorstehenden Figuren gezeigten chirurgischen Verfahren können insbesondere minimalinvasiv sein.

Die in den vorstehenden Figuren gezeigten Implantate sind alle auf dem Prinzip beruhend, dass an ihrer Oberfläche mindestens teilweise thermoplastisches Material vorhanden ist, das in Kontakt hartem Gewebe durch mechanische Schwingungen verflüssigbar ist. Alternativ zu diesem gezeichneten Vorgehen können die Implantate - oder mindestens eines davon - auch eine durch die mechanischen Vibrationen nicht verflüssigbare Hülse mit einer Mehrzahl von Öffnungen aufweisen, wobei im Innern der Hülse verflüssigbares Material vorhanden ist, das beim Implantationsprozess durch die mechanischen Vibrationen verflüssigt und durch die Öffnungen in der Hülse nach aussen gedrückt wird und die porösen Strukturen des harten Gewebes interpenetriert. Auch solche Implantate sind an sich aus dem Stand der Technik bekannt, bspw. aus WO 02/069'817.

Viele weitere chirurgische Verfahren sind denkbar, bei denen die Anwendung der erfindungsgemässen Sonotrode, der erfindungsgemässen Vorrichtung oder des erfindungsgemässen Geräts von Vorteil ist.

Die in den Figuren 10-14 beschriebenen chirurgischen Verfahren können, was weniger bevorzugt ist, auch mit anderen mechanische Schwingungen erzeugenden Geräten und Sonotroden als den hier beschriebenen und beanspruchten realisiert werden.

Bei der Verwendung von erfindungsgemässen Vorrichtungen für chirurgische, zahnmedizinische oder kieferorthopädische Verfahren kann es wünschenswert sein, Gewebeteile von den mechanischen Vibrationen abzuschirmen um Sekundärschäden zu vermeiden. Dies kann durch ein Schutzgehäuse 101 geschehen, wie es sehr schematisch in **Figur 15** illustriert ist. Das Schutzgehäuse kann so ausgebildet sein, dass es durch erste Führungsmittel - hier durch eine erste Öffnung 101.1- direkt am Gehäuse des Vibrationen erzeugenden Geräts aufgesteckt werden kann, so dass die relative Position von Sonotrode 5 und Schutzgehäuse 101 dadurch festgelegt wird und keine aufwändige vibrationsabkoppelnde Lagerung der Sonotrode im Schutzgehäuse notwendig ist. Im Nicht-Betriebszustand kann die Sonotrode relativ zum Gehäuse durch Fixiermittel an Platz gehalten werden, von denen es im Betriebstzustand endkoppelt ist, wobei das Entkoppeln bspw. durch das Aufstecken auf die Sonotrode bewirkt werden kann.

Besonders bevorzugt weist das Schutzgehäuse zweite Führungsmittel auf, mit denen Haltemittel für das Werkzeug, Werkstück oder Zwischenstück geführt werden können. Im sehr schematischen gezeichneten Ausführungsbeispiel werden diese durch eine zweite Öffnung 101.2 gebildet.

## Patentansprüche

1. Vorrichtung (5, 5') zum Umlenken von mechanischen Schwingungen, wobei die Vorrichtung an einer Schwingungs-Aufnahmestelle (5.1) längs einer ersten Achse (6.1) in Schwingung versetzbar ist und eine solche Schwingung an einer Schwingungs-Abgabestelle (5.2) eine Schwingung längs einer zweiten Achse (6.2) erzeugt, wobei die erste und die zweite Achse einen Winkel zueinander bilden, und wobei die Schwingungs-Aufnahmestelle zum Anschliessen eines Schwingungsanregers (2) ausgebildet ist und die Schwingungs-Abgabestelle zum Anschliessen eines Werkzeugs, Werkstücks oder Zwischenstücks (7, 8) ausgebildet ist, **dadurch gekennzeichnet, dass** die Vorrichtung ein Schwingelement (14) aufweist, welches länglich und zwischen zwei Enden gebogen ist und an dem ein Einkoppelpunkt (14.1) und ein Auskoppelpunkt (14.2) angeordnet sind, wobei die Vorrichtung so ausgebildet ist, dass das Schwingelement am Einkoppelpunkt und am Auskoppelpunkt transversal schwingt, wenn die Schwingungs-Aufnahmestelle mit einer Schwingung beaufschlagt wird.

2. Vorrichtung nach Anspruch 1, **dadurch gekennzeichnet, dass** das Schwingelement (14) die transversale Schwingung am Auskoppelpunkt (14.2) bezüglich einer Schwingelement-Achse seitlich abgibt.

3. Vorrichtung nach Anspruch 1 oder 2, **dadurch gekennzeichnet, dass** der Winkel zwischen einer Longitudinalrichtung des Schwingelementes am Einkoppelpunkt (14.1) und einer Longitudinalrichtung des Schwingelementes am Auskoppelpunkt (14.2) mehr als 90° beträgt.

4. Vorrichtung nach einem der vorangehenden Ansprüche, **dadurch gekennzeichnet, dass** das Schwingelement (14) bezüglich eines Neutralpunktes (14.5) zwei Schwingarme (14.3, 14.4) aufweist, wobei der Einkoppelpunkt (14.1) am ersten Schwingarm (14.3) und der Auskoppelpunkt (14.2) am zweiten Schwingarm (14.4) ausgebildet ist, und wobei der Massenschwerpunkt des zweiten Schwingarms (14.4) bezüglich einer Ebene, welche parallel zur ersten Achse (6.1) und senkrecht zu einer durch die erste Achse (6.1) und die zweite Achse (6.2) aufgespannten Achsenebene verläuft, diesseits vom Massenschwerpunkt des ersten Schwingarms (14.3) ausgebildet ist.

5. Vorrichtung nach einem der vorangehenden Ansprüche **dadurch gekennzeichnet, dass** zwischen einem Befestigungselement (11) und dem Einkoppelpunkt (14.1) des Schwingelementes (14) ein Gelenk (15) ausgebildet ist.

6. Vorrichtung nach einem der vorangehenden Ansprüche, **dadurch gekennzeichnet, dass** das Schwingelement (14) in einer Ebene verläuft, wobei vorzugsweise der Auskoppelpunkt (14.2) an einer Aussenseite des Schwingelementes (14) angeordnet ist.

7. Vorrichtung nach einem der vorangehenden Ansprüche, **dadurch gekennzeichnet, dass** der Winkel zwischen der ersten (6.1) und der zweiten Achse (6.2) zwischen 100° und 130° beträgt.

8. Vorrichtung nach einem der vorangehenden Ansprüche, **dadurch gekennzeichnet, dass** der Auskoppelpunkt (14.2) mit der Schwingungs-Abgabestelle (5.2) zusammenfällt.

9. Vorrichtung nach einem der vorangehenden Ansprüche, **dadurch gekennzeichnet, dass** der Einkoppelpunkt (14.1) an einem Ende des Schwingelements (14) und der Auskoppelpunkt (14.2) an einem anderen Ende angeordnet ist.

10. Vorrichtung nach einem der vorangehenden Ansprüche, **gekennzeichnet durch** ein Schutzgehäuse (101) das mindestens das Schwingelement (14) umgibt und die Umgebung von den mechanischen Oszillationen abschirmt.

11. Vorrichtung nach einem der vorangehenden Ansprüche, **dadurch gekennzeichnet, dass** an der Schwingungs-Abgabestelle (5.2) eine Kupplungseinrichtung (16) zum lösbaren Ankoppeln des Werkzeugs, Werkstücks oder Zwischenstücks (7,8) vorhanden ist, wobei die Kupplungseinrichtung (16) vorzugsweise an ein Werkzeug, ein als Implantat oder Präparat ausgebildetes Werkstück (7) oder ein Zwischenstück (8) angepasst ist, wie sie in der Zahnmedizin oder Chirurgie verwendet werden.

12. Einrichtung zum Umlenken von mechanischen Schwingungen, aufweisend eine erste Vorrichtung (5) nach einem der Ansprüche 1 bis 12 und eine zweite Vorrichtung (5') nach einem der Ansprüche 1 bis 12, wobei die Schwingungs-Abgabestelle der ersten Vorrichtung (5) mit der Schwingungs-Aufnahmestelle der zweiten Vorrichtung (5') gekoppelt ist, derart, dass eine transversale Schwingung der ersten Vorrichtung (5) an deren Auskoppelpunkt eine transversale Schwingung der zweiten Vorrichtung (5') an deren Einkoppelpunkt erzeugt.

13. Einrichtung nach Anspruch 12, **dadurch gekennzeichnet, dass** eine Kupplungseinrichtung (16) zwischen der ersten (5) und der zweiten Vorrichtung (5') so ausgebildet ist, dass die zweite Vorrichtung (5') relativ zur ersten Vorrichtung (5) in verschiedenen, sich durch eine Drehung um die zweite Achse (6.2) der ersten Vorrichtung (5) unterscheidenden Positionen anbringbar ist.

14. Ultraschallgerät, aufweisend einen Schwingungsanreger (2), eine Schwingungsanreger-Ansteuerelektronik (4), und eine Sonotrode, **dadurch gekennzeichnet, dass** die Sonotrode als Vorrichtung (5) nach einem der Ansprüche 1 bis 11 ausgebildet ist.

15. Ultraschallgerät nach Anspruch 14, **dadurch gekennzeichnet, dass** die Schwingungsanreger-Ansteuerelektronik (4) so ausgebildet oder programmiert ist, dass die Vorrichtung (5) durch den Schwingungsanreger zu einer Schwingung in einer Frequenz angeregt wird, welche unterhalb ihrer ersten Eigenschwingungsfrequenz liegt.

## Claims

1. Device (5, 5') for deflecting mechanical oscillations, wherein the device at an oscillation receiver location (5.1) may be set into oscillation along a first axis (6.1), and such an oscillation produces an oscillation along a second axis (6.2) at an oscillation output location (5.2), wherein the first axis and the second axis form an angle to one another, and wherein the oscillation receiver location is designed for the connection of an oscillation exciter (2), and the oscillation output location is designed for connecting a tool, work piece or intermediate piece (7, 8), **characterised in that** the device comprises an oscillation element (14) which is elongate and bent between two ends, and on which a coupling-in point (14.1) and a coupling-out point (14.2) are arranged, wherein the device is designed such that the oscillation element oscillates transversally at the coupling-in point and at the coupling-out point, when the oscillation receiver location is subjected to an oscillation.

2. Device according to claim 1, **characterised in that** the oscillation element (14) gives off the transversal oscillation at the coupling-out point (14.2) laterally with respect to an oscillation element axis.

3. Device according to claim 1 or 2, **characterised in that** the angle between a longitudinal direction of the oscillation element at the coupling-in point (14.1) and a longitudinal direction of the oscillation element at the coupling-out point (14.2) is more than 90°.

4. Device according to one of the preceding claims, **characterised in that** the oscillation element (14), with respect to a neutral point (14.5), comprises two oscillation arms (14.3, 14.4), wherein the coupling-in point (14.1) is formed on the first oscillation arm (14.3), and the coupling-out point (14.2) on the second oscillation arm (14.3), and wherein the mass centre of gravity of the second oscillation arm (14.4) with respect to a plane which runs parallel to the first axis (6.1) and perpendicular to an axis plane spanned by the first axis (6.1) and the second axis (6.2), is formed on the same side as the mass centre of gravity of the first oscillation arm (14.3).

5. Device according to one of the preceding claims, **characterised in that** a joint (15) is formed between a fastening element (11) and the coupling-in point (14.1) of the oscillation element (14).

6. Device according to one of the preceding claims, **characterised in that** the oscillation element (14) runs in a plane, wherein the coupling-out point (14.2) is preferably arranged on an outer side of the oscillation element (14).

7. Device according to one of the preceding claims, **characterised in that** the angle between the first (6.1) and the second axis (6.2) is between 100° and 130°.

8. Device according to one of the preceding claims, **characterised in that** the coupling-out point (14.2) coincides with the oscillation output location (5.2).

9. Device according to one of the preceding claims, **characterised in that** the coupling-in point (14.1) is arranged at one end of the oscillation element (14), and the coupling-out point (14.2) is arranged at another end.

10. Device according to one of the preceding claims, **characterised in that** a protective housing (101) surrounds at least the oscillation element (14) and shields the surroundings from the mechanical oscillations.

11. Device according to one of the preceding claims, **characterised in that** a coupling arrangement (16) for the releasable coupling of the tool, work piece or intermediate piece (7, 8) is present at the oscillation output location (5.2) wherein the coupling arrangement (16) is adapted to a tool, to a work piece (7) designed as an implant or preparation or to an intermediate piece (8), as is applied in dentistry or surgery.

12. Appliance for deflecting mechanical oscillations, comprising a first device (5) according to one of the claims 1 to 12, and a second device (5') according to one of the claims 1 to 12, wherein the oscillation output location of the first device (5) is coupled to the oscillation receiver location of the second device (5'), in a manner such that a transversal oscillation of the first device (5) at its coupling-out point produces a transversal oscillation of the second device (5') at its coupling-in point.

13. Appliance according to claim 12, **characterised in that** a coupling arrangement (16) between the first (5) and the second device (5') is designed such that the second device (5') may be attached relative to the first device (5) in different positions differing by a rotation about the second axis (6.2) of the first device (5).

14. Ultrasound apparatus, comprising an oscillation exciter (2), oscillation exciter activation electronics (4) and a sonotrode, **characterised in that** the sonotrode is designed as a device (5) according to one of the claims 1 to 11.

15. Ultrasound apparatus according to claim 14, **characterised in that** the oscillation exciter activation electronics (4) are designed or programmed such that the device (5) is excited by the oscillation exciter into an oscillation in a frequency which lies below its first natural oscillation frequency.

## Revendications

1. Dispositif (5, 5') de transfert d'oscillations mécaniques,
le dispositif pouvant être mis en oscillation le long d'un premier axe (6.1) en un emplacement (5.1) de réception d'oscillations et cette oscillation produisant en un emplacement (5.2) de transfert d'oscillations une oscillation le long d'un deuxième axe (6.2),
le premier et le deuxième axe formant un angle l'un avec l'autre et l'emplacement de réception d'oscillations étant configuré pour être raccordé à un excitateur d'oscillations (2),
l'emplacement de transfert d'oscillations étant configuré pour être raccordé à un outil, une pièce ou une pièce intermédiaire (7, 8),
**caractérisé en ce que**
le dispositif présente un élément oscillant (14) qui est allongé, courbé entre deux extrémités et sur lequel un point d'injection (14.1) et un point d'extraction (14.2) sont disposés et
**en ce que** le dispositif est configuré de telle sorte que l'élément oscillant oscille transversalement sur le point d'injection et sur le point d'extraction lorsqu'une oscillation est appliquée sur l'emplacement de réception d'oscillations.

2. Dispositif selon la revendication 1, **caractérisé en ce que** l'élément oscillant (14) transfère l'oscillation transversale sur le point d'extraction (14.2) latéralement par rapport à un axe de l'élément oscillant.

3. Dispositif selon les revendications 1 ou 2,
**caractérisé en ce que** l'angle entre la direction longitudinale de l'élément oscillant sur le point d'injection (14.1) et la direction longitudinale de l'élément oscillant au point d'extraction (14.2) est supérieur à 90°.

4. Dispositif selon l'une des revendications précédentes, **caractérisé en ce que** l'élément oscillant (14) présente deux bras oscillants (14.3, 14.4) situés de part et d'autre d'un point neutre (14.5), le point d'injection (14.1) étant formé sur le premier bras oscillant (14.3) et le point d'extraction (14.2) sur le deuxième bras oscillant (14.4), le centre de masse du deuxième bras oscillant (14.4) et le centre de masse du premier bras oscillant (14.3) étant formés sue le même côté d'un plan qui s'étend parallèlement au premier axe (6.1) et perpendiculairement au plan sous-tendu par le premier axe (6.1) et le deuxième axe (6.2).

5. Dispositif selon l'une des revendications précédentes, **caractérisé en ce qu'**une articulation (15) est formée entre un point de fixation (11) et le point d'injection (14.1) de l'élément oscillant (14).

6. Dispositif selon l'une des revendications précédentes, **caractérisé en ce que** l'élément oscillant (14) s'étend dans un plan et **en ce que** le point d'extraction (14.2) est disposé de préférence sur le côté extérieur de l'élément oscillant (14).

7. Dispositif selon l'une des revendications précédentes, **caractérisé en ce que** l'angle entre le premier axe (6.1) et le deuxième axe (6.2) est compris entre 100° et 130°.

8. Dispositif selon l'une des revendications précédentes, **caractérisé en ce que** le point d'extraction (14.2) coïncide avec l'emplacement (5.2) de transfert d'oscillations.

9. Dispositif selon l'une des revendications précédentes, **caractérisé en ce que** le point d'injection (14.1) est disposé à une extrémité de l'élément oscillant (14) et **en ce que** le point d'extraction (14.2) est disposé à son autre extrémité.

10. Dispositif selon l'une des revendications précédentes, **caractérisé par** un boîtier de protection (101) qui entoure au moins l'élément oscillant (14) et qui protège l'environnement d'oscillations mécaniques.

11. Dispositif selon l'une des revendications précédentes, **caractérisé en ce qu'**un système d'accouplement (16) qui permet un accouplement libérable de l'outil, de la pièce ou de la pièce intermédiaire (7, 8) est prévu à l'emplacement (5.2) de transfert d'oscillations, le système d'accouplement (16) étant de préférence adapté à un outil, à une pièce (7) configurée comme implant ou préparation ou à une pièce intermédiaire (8) qui sont utilisées en médecine dentaire ou en chirurgie.

12. Système de transfert d'oscillations mécaniques qui présente un premier dispositif (5) selon l'une des revendications 1 à 12 et un deuxième dispositif (5') selon l'une des revendications 1 à 12, l'emplacement de transfert d'oscillations du premier dispositif (5) étant accouplé à l'emplacement de réception d'oscillations du deuxième dispositif (5') de telle sorte qu'une oscillation transversale du premier dispositif (5) produise sur son point de transfert une oscillation transversale du point d'injection du deuxième dispositif (5').

13. Système selon la revendication 12, **caractérisé en ce qu'**un système d'accouplement (16) est formé entre le premier dispositif (5) et le deuxième dispositif (5') de telle sorte que le deuxième dispositif (5') puisse être amené en différentes positions qui se distinguent par une rotation autour du deuxième axe (6.2) du premier dispositif (5).

14. Appareil à ultrasons présentant un excitateur d'oscillations (2), une électronique (4) de commande de l'excitateur d'oscillations et une sonotrode, **caractérisé en ce que** la sonotrode est configurée comme dispositif (5) selon l'une des revendications 1 à 11.

15. Appareil à ultrasons selon la revendication 14, **caractérisé en ce que** l'électronique (4) de commande de l'excitateur d'oscillations est configurée ou programmée de telle sorte que l'excitateur d'oscillations excite le dispositif (5) à osciller à une première fréquence plus basse que sa première fréquence propre d'oscillation.
